# EUROPÄISCHE PATENTSCHRIFT

(11) **EP 1 319 022 B1**
(45) Veröffentlichungstag und Bekanntmachung des Hinweises auf die Patenterteilung: **31.03.2010**
(21) Anmeldenummer: 01951650.9
(22) Anmeldetag: 05.07.2001
(51) Int. Cl.: C07K 14/47

(54) **BREIT EINSETZBARES VERFAHREN ZUR IDENTIFIZIERUNG VON MODULATOREN VON G-PROTEIN GEKOPPELTEN REZEPTOREN**
METHOD WITH A WIDE RANGE OF APPLICATIONS, FOR IDENTIFYING MODULATORS OF G-PROTEIN-COUPLED RECEPTORS
PROCEDE A LARGE CHAMP D'APPLICATION PERMETTANT D'IDENTIFIER DES MODULATEURS DE RECEPTEURS COUPLES A LA PROTEINE G

(30) Priorität: 08.07.2000 DE 10033353
(43) Veröffentlichungstag der Anmeldung: 18.06.2003
(73) Patentinhaber: Sanofi-Aventis Deutschland GmbH, 65929 Frankfurt am Main (DE)
(72) Erfinder: KOSTENIS, Evi, 60594 Frankfurt am Main (DE)
(86) Internationale Anmeldenummer: PCT/EP2001/007667
(87) Internationale Veröffentlichungsnummer: WO 2002/004665

(56) Entgegenhaltungen:
- WO-A-97/48820
- WO-A-99/05177
- COWARD P ET AL: "CHIMERIC G PROTEINS ALLOW A HIGH-THROUGHPUT SIGNALING ASSAY OF GI-COUPLED RECEPTORS" ANALYTICAL BIOCHEMISTRY, ACADEMIC PRESS, SAN DIEGO, CA, US, Bd. 270, Nr. 2, 1. Juni 1999 (1999-06-01), Seiten 242-248, XP000984715 ISSN: 0003-2697
- KOSTENIS EVI ET AL: "The N-terminal extension of G-alpha-q is critical for constraining the selectivity of receptor coupling." JOURNAL OF BIOLOGICAL CHEMISTRY, Bd. 272, Nr. 31, 1997, Seiten 19107-19110, XP002210249 ISSN: 0021-9258
- CONKLIN B R: ET AL: "Carboxyl-terminal mutations of Gq alpha and Gs alpha that alter the fidelity of receptor activation" MOLECULAR PHARMACOLOGY, BALTIMORE, MD, US, Bd. 50, Nr. 4, 1. Oktober 1996 (1996-10-01), Seiten 885-890, XP002090508 ISSN: 0026-895X
- MIGEON JACQUES C ET AL: "Regulation of cAMP-mediated gene transcription by wild type and mutated G-protein alpha subunits: Inhibition of adenylyl cyclase activity by muscarinic receptor-activated constitutively activated G-o-alpha." JOURNAL OF BIOLOGICAL CHEMISTRY, Bd. 269, Nr. 46, 1994, Seiten 29146-29152, XP002210250 ISSN: 0021-9258
- KOSTENIS E.: 'Potentiation of GPCR-signaling via membrane targeting of G protein alpha subunits' JOURNAL OF RECEPTORS AND SIGNAL TRANSDUCTION Bd. 22, Nr. 1-4, 2002, Seiten 267 - 281, XP009097011

### SEQUENZPROTOKOLL

<110> Aventis Pharma Deutschland GmbH
<120> Breit einsetzbares Verfahren zur Identifizierung von Modulatoren von G-Protein gekoppelten Rezeptoren
<130> AVE D-2000/A033
<140>
   <141>
<160> 10
<170> PatentIn Ver. 2.1
<210> 1
   <211> 1080
   <212> DNA
   <213> Mus musculus
<400> 1
   atgactctgg agtccatcat ggcgtgctgc ctgagcgagg aggccaagga agcccggcgg 60
   atcaacgacg agatcgagcg gcacgtccgc agggacaagc gggacgcccg ccgggagctc 120
   aagctgctgc tgctcgggac aggagagagt ggcaagagta cgtttatcaa gcagatgaga 180
   atcatccatg ggtcaggata ctctgatgaa gataaaaggg gcttcaccaa gctggtgtat 240
   cagaacatct tcacggccat gcaggccatg atcagagcca tggacacact caagatccca 300
   tacaagtatg agcacaataa ggctcatgca caattagttc gagaagttga tgtggagaag 360
   gtgtctgctt ttgagaatcc atatgtagat gcaataaaga gtttatggaa tgatcctgga 420
   atccaggaat gctatgatag acgacgagaa tatcaattat ctgactctac caaatactat 480
   cttaatgact tggaccgcgt agctgaccct gcctacctgc ctacgcaaca agatgtgctt 540
   agagttcgag tccccaccac agggatcatc gaatacccct ttgacttaca aagtgtcatt 600
   ttcagaatgg tcgatgtagg gggccaaagg tcagagagaa gaaaatggat acactgcttt 660
   gaaaatgtca cctctatcat gtttctagta gcgcttagtg aatatgatca agttctcgtg 720
   gagtcagaca atgagaaccg aatggaggaa agcaaggctc tctttagaac aattatcaca 780
   tacccctggt tccagaactc ctcggttatt ctgttcttaa acaagaaaga tcttctagag 840
   gagaaaatca tgtattccca tctagtcgac tacttcccag aatatgatgg accccagaga 900
   gatgcccagg cagcccgaga attcattctg aagatgttcg tggacctgaa cccagacagt 960
   gacaaaatta tctactccca cttcacgtgc gccacagaca ccgagaatat ccgctttgtc 1020
   tttgctgccg tcaaggacac catcctccag ttgaacctga aggagtacaa tctggtctaa 1080
<210> 2
   <211> 359
   <212> PRT
   <213> Mus musculus
<400> 2
<210> 3
   <211> 1062
   <212> DNA
   <213> Mus musculus
<400> 3
   atggggtgct gcctgagcga ggaggccaag gaagcccggc ggatcaacga cgagatcgag 60
   cggcacgtcc gcagggacaa gcgggacgcc cgccgggagc tcaagctgct gctgctcggg 120
   acaggagaga gtggcaagag tacgtttatc aagcagatga gaatcatcca tgggtcagga 180
   tactctgatg aagataaaag gggcttcacc aagctggtgt atcagaacat cttcacggcc 240
   atgcaggcca tgatcagagc catggacaca ctcaagatcc catacaagta tgagcacaat 300
   aaggctcatg cacaattagt tcgagaagtt gatgtggaga aggtgtctgc ttttgagaat 360
   ccatatgtag atgcaataaa gagtttatgg aatgatcctg gaatccagga atgctatgat 420
   agacgacgag aatatcaatt atctgactct accaaatact atcttaatga cttggaccgc 480
   gtagctgacc ctgcctacct gcctacgcaa caagatgtgc ttagagttcg agtccccacc 540
   acagggatca tcgaataccc ctttgactta caaagtgtca ttttcagaat ggtcgatgta 600
   gggggccaaa ggtcagagag aagaaaatgg atacactgct ttgaaaatgt cacctctatc 660
   atgtttctag tagcgcttag tgaatatgat caagttctcg tggagtcaga caatgagaac 720
   cgaatggagg aaagcaaggc tctctttaga acaattatca catacccctg gttccagaac 780
   tcctcggtta ttctgttctt aaacaagaaa gatcttctag aggagaaaat catgtattcc 840
   catctagtcg actacttccc agaatatgat ggaccccaga gagatgccca ggcagcccga 900
   gaattcattc tgaagatgtt cgtggacctg aacccagaca gtgacaaaat tatctactcc 960
   cacttcacgt gcgccacaga caccgagaat atccgctttg tctttgctgc cgtcaaggac 1020
   accatcctcc agttgaacct gaaggagtgt ggcctcttct aa                    1062
<210> 4
   <211> 353
   <212> PRT
   <213> Mus musculus
<400> 4
   Met Gly Cys Cys Leu Ser Glu Glu Ala Lys Glu Ala Arg Arg Ile Asn
    1               5                  10                  15
   Asp Glu Ile Glu Arg His Val Arg Arg Asp Lys Arg Asp Ala Arg Arg
               20                  25                  30
   Glu Leu Lys Leu Leu Leu Leu Gly Thr Gly Glu Ser Gly Lys Ser Thr
           35                  40                  45
   Phe Ile Lys Gln Met Arg Ile Ile His Gly Ser Gly Tyr Ser Asp Glu
       50                  55                  60
   Asp Lys Arg Gly Phe Thr Lys Leu Val Tyr Gln Asn Ile Phe Thr Ala
   65                  70                  75                  80
   Met Gln Ala Met Ile Arg Ala Met Asp Thr Leu Lys Ile Pro Tyr Lys
                   85                  90                  95
   Tyr Glu His Asn Lys Ala His Ala Gln Leu Val Arg Glu Val Asp Val
               100                 105                 110
   Glu Lys Val Ser Ala Phe Glu Asn Pro Tyr Val Asp Ala Ile Lys Ser
           115                 120                 125
   Leu Trp Asn Asp Pro Gly Ile Gln Glu Cys Tyr Asp Arg Arg Arg Glu
       130                 135                 140
   Tyr Gln Leu Ser Asp Ser Thr Lys Tyr Tyr Leu Asn Asp Leu Asp Arg
   145                 150                 155                 160
   Val Ala Asp Pro Ala Tyr Leu Pro Thr Gln Gln Asp Val Leu Arg Val
                   165                 170                 175
   Arg Val Pro Thr Thr Gly Ile Ile Glu Tyr Pro Phe Asp Leu Gln Ser
               180                 185                 190
   Val Ile Phe Arg Met Val Asp Val Gly Gly Gln Arg Ser Glu Arg Arg
           195                 200                 205
   Lys Trp Ile His Cys Phe Glu Asn Val Thr Ser Ile Met Phe Leu Val
       210                 215                 220
   Ala Leu Ser Glu Tyr Asp Gln Val Leu Val Glu Ser Asp Asn Glu Asn
   225 230 235 240
   Arg Met Glu Glu Ser Lys Ala Leu Phe Arg Thr Ile Ile Thr Tyr Pro
   245 250 255
   Trp Phe Gln Asn Ser Ser Val Ile Leu Phe Leu Asn Lys Lys Asp Leu
   260 265 270
   Leu Glu Glu Lys Ile Met Tyr Ser His Leu Val Asp Tyr Phe Pro Glu
   275 280 285
   Tyr Asp Gly Pro Gln Arg Asp Ala Gln Ala Ala Arg Glu Phe Ile Leu
   290 295 300
   Lys Met Phe Val Asp Leu Asn Pro Asp Ser Asp Lys Ile Ile Tyr Ser
   305 310 315 320
   His Phe Thr Cys Ala Thr Asp Thr Glu Asn Ile Arg Phe Val Phe Ala
   325 330 335
   Ala Val Lys Asp Thr Ile Leu Gln Leu Asn Leu Lys Glu Cys Gly Leu
   340 345 350
   Phe
<210> 5
   <211> 1062
   <2-12> DNA
   <213> Mus musculus
<400> 5
   atggcgtgct gcctgagcga ggaggccaag gaagcccggc ggatcaacga cgagatcgag 60
   cggcacgtcc gcagggacaa gcgggacgcc cgccgggagc tcaagctgct gctgctcggg 120
   acaggagaga gtggcaagag tacgtttatc aagcagatga gaatcatcca tgggtcagga 180
   tactctgatg aagataaaag gggcttcacc aagctggtgt atcagaacat cttcacggcc 240
   atgcaggcca tgatcagagc catggacaca ctcaagatcc catacaagta tgagcacaat 300
   aaggctcatg cacaattagt tcgagaagtt gatgtggaga aggtgtctgc ttttgagaat 360
   ccatatgtag atgcaataaa gagtttatgg aatgatcctg gaatccagga atgctatgat 420
   agacgacgag aatatcaatt atctgactct accaaatact atcttaatga cttggaccgc 480
   gtagctgacc ctgcctacct gcctacgcaa caagatgtgc ttagagttcg agtccccacc 540
   acagggatca tcgaataccc ctttgactta caaagtgtca ttttcagaat ggtcgatgta 600
   gggggccaaa ggtcagagag aagaaaatgg atacactgct ttgaaaatgt cacctctatc 660
   atgtttctag tagcgcttag tgaatatgat caagttctcg tggagtcaga caatgagaac 720
   cgaatggagg aaagcaaggc tctctttaga acaattatca catacccctg gttccagaac 780
   tcctcggtta ttctgttctt aaacaagaaa gatcttctag aggagaaaat catgtattcc 840
   catctagtcg actacttccc agaatatgat ggaccccaga gagatgccca ggcagcccga 900
   gaattcattc tgaagatgtt cgtggacctg aacccagaca gtgacaaaat tatctactcc 960
   cacttcacgt gcgccacaga caccgagaat atccgctttg tctttgctgc cgtcaaggac 1020
   accatcctcc agttgaacct gaaggagtgt ggcctcttct aa                    1062
<210> 6
   <211> 353
   <212> PRT
   <213> Mus musculus
<400> 6
   Met Ala Cys Cys Leu Ser Glu Glu Ala Lys Glu Ala Arg Arg Ile Asn
   1 5 10 15
   Asp Glu Ile Glu Arg His Val Arg Arg Asp Lys Arg Asp Ala Arg Arg
   20 25 30
   Glu Leu Lys Leu Leu Leu Leu Gly Thr Gly Glu Ser Gly Lys Ser Thr
   35 40 45
   Phe Ile Lys Gln Met Arg Ile Ile His Gly Ser Gly Tyr Ser Asp Glu
   50 55 60
   Asp Lys Arg Gly Phe Thr Lys Leu Val Tyr Gln Asn Ile Phe Thr Ala
   65 70 75 80
   Met Gln Ala Met Ile Arg Ala Met Asp Thr Leu Lys Ile Pro Tyr Lys
   85 90 95
   Tyr Glu His Asn Lys Ala His Ala Gln Leu Val Arg Glu Val Asp Val
   100 105 110
   Glu Lys Val Ser Ala Phe Glu Asn Pro Tyr Val Asp Ala Ile Lys Ser
   115 120 125
   Leu Trp Asn Asp Pro Gly Ile Gln Glu Cys Tyr Asp Arg Arg Arg Glu
   130 135 140
   Tyr Gln Leu Ser Asp Ser Thr Lys Tyr Tyr Leu Asn Asp Leu Asp Arg
   145 150 155 160
   Val Ala Asp Pro Ala Tyr Leu Pro Thr Gln Gln Asp Val Leu Arg Val
   165 170 175
   Arg Val Pro Thr Thr Gly Ile Ile Glu Tyr Pro Phe Asp Leu Gln Ser
   180 185 190
   Val Ile Phe Arg Met Val Asp Val Gly Gly Gln Arg Ser Glu Arg Arg
   195 200 205
   Lys Trp Ile His Cys Phe Glu Asn Val Thr Ser Ile Met Phe Leu Val
   210 215 220
   Ala Leu Ser Glu Tyr Asp Gln Val Leu Val Glu Ser Asp Asn Glu Asn
   225 230 235 240
   Arg Met Glu Glu Ser Lys Ala Leu Phe Arg Thr Ile Ile Thr Tyr Pro
   245 250 255
   Trp Phe Gln Asn Ser Ser Val Ile Leu Phe Leu Asn Lys Lys Asp Leu
   260 265 270
   Leu Glu Glu Lys Ile Met Tyr Ser His Leu Val Asp Tyr Phe Pro Glu
   275 280 285
   Tyr Asp Gly Pro Gln Arg Asp Ala Gln Ala Ala Arg Glu Phe Ile Leu
   290 295 300
   Lys Met Phe Val Asp Leu Asn Pro Asp Ser Asp Lys Ile Ile Tyr Ser
   305 310 315 320
   His Phe Thr Cys Ala Thr Asp Thr Glu Asn Ile Arg Phe Val Phe Ala
   325 330 335
   Ala Val Lys Asp Thr Ile Leu Gln Leu Asn Leu Lys Glu Cys Gly Leu
   340 345 350
   Phe
<210> 7
   <211> 1062
   <212> DNA
   <213> Mus musculus
<400> 7
   atggcgtgct gcctgagcga ggaggccaag gaagcccggc ggatcaacga cgagatcgag 60
   cggcacgtcc gcagggacaa gcgggacgcc cgccgggagc tcaagctgct gctgctcggg 120
   acaggagaga gtggcaagag tacgtttatc aagcagatga gaatcatcca tgggtcagga 180
   tactctgatg aagataaaag gggcttcacc aagctggtgt atcagaacat cttcacggcc 240
   atgcaggcca tgatcagagc catggacaca ctcaagatcc catacaagta tgagcacaat 300
   aaggctcatg cacaattagt tcgagaagtt gatgtggaga aggtgtctgc ttttgagaat 360
   ccatatgtag atgcaataaa gagtttatgg aatgatcctg gaatccagga atgctatgat 420
   agacgacgag aatatcaatt atctgactct accaaatact atcttaatga cttggaccgc 480
   gtagctgacc ctgcctacct gcctacgcaa caagatgtgc ttagagttcg agtccccacc 540
   acagggatca tcgaataccc ctttgactta caaagtgtca ttttcagaat ggtcgatgta 600
   gggggccaaa ggtcagagag aagaaaatgg atacactgct ttgaaaatgt cacctctatc 660
   atgtttctag tagcgcttag tgaatatgat caagttctcg tggagtcaga caatgagaac 720
   cgaatggagg aaagcaaggc tctctttaga acaattatca catacccctg gttccagaac 780
   tcctcggtta ttctgttctt aaacaagaaa gatcttctag aggagaaaat catgtattcc 840
   catctagtcg actacttccc agaatatgat ggaccccaga gagatgccca ggcagcccga 900
   gaattcattc tgaagatgtt cgtggacctg aacccagaca gtgacaaaat tatctactcc 960
   cacttcacgt gcgccacaga caccgagaat atccgctttg tctttgctgc cgtcaaggac 1020
   accatcctcc agttgaacct gaaggagtgt ggcctcttct aa                    1062
<210> 8
   <211> 353
   <212> PRT
   <213> Mus musculus
<400> 8
   Met Ala Cys Cys Leu Ser Glu Glu Ala Lys Glu Ala Arg Arg Ile Asn
    1               5                  10                  15
   Asp Glu Ile Glu Arg His Val Arg Arg Asp Lys Arg Asp Ala Arg Arg
               20                  25                  30
   Glu Leu Lys Leu Leu Leu Leu Gly Thr Gly Glu Ser Gly Lys Ser Thr
           35                  40                  45
   Phe Ile Lys Gln Met Arg Ile Ile His Gly Ser Gly Tyr Ser Asp Glu
       50                  55                  60
   Asp Lys Arg Gly Phe Thr Lys Leu Val Tyr Gln Asn Ile Phe Thr Ala
   65                  70                  75                  80
   Met Gln Ala Met Ile Arg Ala Met Asp Thr Leu Lys Ile Pro Tyr Lys
                   85                  90                  95
   Tyr Glu His Asn Lys Ala His Ala Gln Leu Val Arg Glu Val Asp Val
               100                 105                 110
   Glu Lys Val Ser Ala Phe Glu Asn Pro Tyr Val Asp Ala Ile Lys Ser
           115                 120                 125
   Leu Trp Asn Asp Pro Gly Ile Gln Glu Cys Tyr Asp Arg Arg Arg Glu
       130                 135                 140
   Tyr Gln Leu Ser Asp Ser Thr Lys Tyr Tyr Leu Asn Asp Leu Asp Arg
   145                 150                 155                 160
   Val Ala Asp Pro Ala Tyr Leu Pro Thr Gln Gln Asp Val Leu Arg Val
                   165                 170                 175
   Arg Val Pro Thr Thr Gly Ile Ile Glu Tyr Pro Phe Asp Leu Gln Ser
               180                 185                 190
   Val Ile Phe Arg Met Val Asp Val Gly Gly Gln Arg Ser Glu Arg Arg
           195                 200                 205
   Lys Trp Ile His Cys Phe Glu Asn Val Thr Ser Ile Met Phe Leu Val
       210                 215                 220
   Ala Leu Ser Glu Tyr Asp Gln Val Leu Val Glu Ser Asp Asn Glu Asn
   225                 230                 235                 240
   Arg Met Glu Glu Ser Lys Ala Leu Phe Arg Thr Ile Ile Thr Tyr Pro
                   245                 250                 255
   Trp Phe Gln Asn Ser Ser Val Ile Leu Phe Leu Asn Lys Lys Asp Leu
               260                 265                 270
   Leu Glu Glu Lys Ile Met Tyr Ser His Leu Val Asp Tyr Phe Pro Glu
           275                 280                 285
   Tyr Asp Gly Pro Gln Arg Asp Ala Gln Ala Ala Arg Glu Phe Ile Leu
       290                 295                 300
   Lys Met Phe Val Asp Leu Asn Pro Asp Ser Asp Lys Ile Ile Tyr Ser
   305                 310                 315                 320
   His Phe Thr Cys Ala Thr Asp Thr Glu Asn Ile Arg Phe Val Phe Ala
                   325                 330                 335
   Ala Val Lys Asp Thr Ile Leu Gln Leu Asn Leu Lys Glu Cys Gly Leu
               340                 345                 350
   Phe
<210> 9
   <211> 1128
   <212> DNA
   <213> Mus musculus
<400> 9
   gccatggccc gctcgctgac ctggcgctgc tgcccctggt gcctgacgga ggatgagaag 60
   gccgccgccc gggtggacca ggagatcaac aggatcctct tggagcagaa gaagcaggac 120
   cgcggggagc tgaagctgct gcttttgggc ccaggcgaga gcgggaagag caccttcatc 180
   aagcagatgc ggatcatcca cggcgccggc tactcggagg aggagcgcaa gggcttccgg 240
   cccctggtct accagaacat cttcgtgtcc atgcgggcca tgatcgaggc catggagcgg 300
   ctgcagattc cattcagcag gcccgagagc aagcaccacg ctagcctggt catgagccag 360
   gacccctata aagtgaccac gtttgagaag cgctacgctg cggccatgca gtggctgtgg 420
   agggatgccg gcatccgggc ctgctatgag cgtcggcggg aattccacct gctcgattca 480
   gccgtgtact acctgtccca cctggagcgc atcaccgagg agggctacgt ccccacagct 540
   caggacgtgc tccgcagccg catgcccacc actggcatca acgagtactg cttctccgtg 600
   cagaaaacca acctgcggat cgtggacgtc gggggccaga agtcagagcg taagaaatgg 660
   atccattgtt tcgagaacgt gatcgccctc atctacctgg cctcactgag tgaatacgac 720
   cagtgcctgg aggagaacaa ccaggagaac cgcatgaagg agagcctcgc attgtttggg 780
   actatcctgg aactaccctg gttcaaaagc acatccgtca tcctctttct caacaaaacc 840
   gacatcctgg aggagaaaat ccccacctcc cacctggcta cctatttccc cagtttccag 900
   ggccctaagc aggatgctga ggcagccaag aggttcatcc tggacatgta cacgaggatg 960
   tacaccgggt gcgtggacgg ccccgagggc agcaagaagg gcgcacgatc ccgacgcctt 1020
   ttcagccact acacatgtgc cacagacaca cagaacatcc gcaaggtctt caaggacgtg 1080
   cgggactcgg tgctcgcccg ctacctggac gagatcaacc tgctgtga              1128
<210> 10
   <211> 374
   <212> PRT
   <213> Mus musculus
<400> 10
   Met Ala Arg Ser Leu Thr Trp Arg Cys Cys Pro Trp Cys Leu Thr Glu
   1               5                   10                  15
   Asp Glu Lys Ala Ala Ala Arg Val Asp Gln Glu Ile Asn Arg Ile Leu
               20                  25                  30
   Leu Glu Gln Lys Lys Gln Asp Arg Gly Glu Leu Lys Leu Leu Leu Leu
           35                  40                  45
   Gly Pro Gly Glu Ser Gly Lys Ser Thr Phe Ile Lys Gln Met Arg Ile
       50                  55                  60
   Ile His Gly Ala Gly Tyr Ser Glu Glu Glu Arg Lys Gly Phe Arg Pro
   65                  70                  75                  80
   Leu Val Tyr Gln Asn Ile Phe Val Ser Met Arg Ala Met Ile Glu Ala
                   85                  90                  95
   Met Glu Arg Leu Gln Ile Pro Phe Ser Arg Pro Glu Ser Lys His His
               100                 105                 110
   Ala Ser Leu Val Met Ser Gln Asp Pro Tyr Lys Val Thr Thr Phe Glu
           115                 120                 125
   Lys Arg Tyr Ala Ala Ala Met Gln Trp Leu Trp Arg Asp Ala Gly Ile
       130                 135                 140
   Arg Ala Cys Tyr Glu Arg Arg Arg Glu Phe His Leu Leu Asp Ser Ala
   145                 150                 155                 160
   Val Tyr Tyr Leu Ser His Leu Glu Arg Ile Thr Glu Glu Gly Tyr Val
                   165                 170                 175
   Pro Thr Ala Gln Asp Val Leu Arg Ser Arg Met Pro Thr Thr Gly Ile
               180                 185                 190
   Asn Glu Tyr Cys Phe Ser Val Gln Lys Thr Asn Leu Arg Ile Val Asp
           195                 200                 205
   Val Gly Gly Gln Lys Ser Glu Arg Lys Lys Trp Ile His Cys Phe Glu
       210                 215                 220
   Asn Val Ile Ala Leu Ile Tyr Leu Ala Ser Leu Ser Glu Tyr Asp Gln
   225                 230                 235                 240
   Cys Leu Glu Glu Asn Asn Gln Glu Asn Arg Met Lys Glu Ser Leu Ala
                   245                 250                 255
   Leu Phe Gly Thr Ile Leu Glu Leu Pro Trp Phe Lys Ser Thr Ser Val
               260                 265                 270
   Ile Leu Phe Leu Asn Lys Thr Asp Ile Leu Glu Glu Lys Ile Pro Thr
           275                 280                 285
   Ser His Leu Ala Thr Tyr Phe Pro Ser Phe Gln Gly Pro Lys Gln Asp
       290                 295                 300
   Ala Glu Ala Ala Lys Arg Phe Ile Leu Asp Met Tyr Thr Arg Met Tyr
   305                 310                 315                 320
   Thr Gly Cys Val Asp Gly Pro Glu Gly Ser Lys Lys Gly Ala Arg Ser
                   325                 330                 335
   Arg Arg Leu Phe Ser His Tyr Thr Cys Ala Thr Asp Thr Gln Asn Ile
               340                 345                 350
   Arg Lys Val Phe Lys Asp Val Arg Asp Ser Val Leu Ala Arg Tyr Leu
   355 360 365
   Asp Glu Ile Asn Leu Leu
   370

## Beschreibung

Die Erfindung bezieht sich auf ein breit einsetzbares Verfahren zur Identifizierung von chemischen Verbindungen, welche G-Protein gekoppelten Rezeptoren modulieren, mittels neuer Hybrid-G-Proteine mit sehr weiter Rezeptorspezifität sowie auf chemischen Verbindungen, welche durch ein solches Verfahren identifiziert werden können.

G-Protein gekoppelte Rezeptoren (GPCR) spielen eine wichtige Rolle bei einer Vielzahl physiologischer Prozesse. Sie stellen eine der wichtigsten, bisher bekannten Proteinfamilien dar und man nimmt an, daß im menschlichen Genom etwa 1000 Gene für diese Rezeptorklasse kodieren. GPCRs haben eine charakteristische Struktur: Es handelt sich um Peptidfäden, die sich siebenfach in Form von α-Helices durch die Phospholipid-Doppelschicht der Zellmembran winden, wobei sie sich kreisförmig anordnen. Man schätzt, daß etwa 60 % der gegenwärtig verfügbaren verschreibungspflichtigen Arzneimittel an GPCRs binden. Dies unterstreicht die bedeutende Rolle dieser Rezeptorklasse für die arzneimittelforschende Industrie.
Alle G-Protein gekoppelten Rezeptoren funktionieren nach einem gemeinsamen Grundmuster: die Bindung eines extrazellulären Liganden führt zu einer Konformationsänderung des Rezeptorproteins so, daß dieses Kontakt mit einem G-Protein aufnehmen kann. Die an der zytoplasmatischen Seite der Plasmamembran gelegenen G-Proteine sind die Vermittler des extrazellulären Signals in das Zellinnere und können dort verschiedene Reaktionen auslösen.
GPCRs stellen die bisher bedeutendsten therapeutischen Zielproteine dar. Schätzungsweise 40% der vom Arzt verordneten Arzneimittel wirken als Agonisten oder Antagonisten von GPCRs. Aufgrund der Größe und Bedeutung der Proteinfamilie und angesichts Tatsache, daß für viele GPCRs noch keine chemischen Bindepartner bekannt sind (orphan GPCRs), ist davon auszugehen, daß diese Rezeptorklasse in Zukunft eines der wichtigsten Reservoirs für geeignete Zielproteine bei der Suche nach neuen Arzneistoffen sein wird

GPCRs sind integrale Membranproteine. Sie übertragen ein Signal vermittelt über einen meist hydrophilen Signalstoff, der auf der äußeren Seite der Zelle gebunden wird, über eine Familie von Guanin-Nucleotid-bindenden Proteinen, sogenannten G-Proteinen, ins Zellinnere. Dabei lösen sie in Abhängigkeit von der Spezifität des Rezeptors und der dadurch aktivierten G-Proteine verschiedene Signaltransduktionswege aus. Je nach Typ des Rezeptors werden verschiedene Wirkungen hervorgerufen, die alle die Bildung von "second messengers" zur Folge haben. So kann über die Aktivierung einer membranständigen Adenylat-Cyclase der intracelluläre cAMP Spiegel ansteigen bzw. bei einer Hemmung abfallen. Die Stimulierung einer cGMP-spezifischen Phosphodiesterase kann zur Absenkung des cGMP-Spiegels führen. Weiterhin kann das aktivierte G-Protein über die Bindung an einen lonenkanal beispielweise zum Anstieg von Ca²⁺- oder K⁺ -Ionen führen. Weiterhin kann über ein aktiviertes G-Protein die Aktivierung einer Phospholipase und damit die Bildung von Inositol-1,4,5-trisphosphat und Diacylglycerolbewirkt werden. Diese wiederum führen entweder zum Ca²⁺-Anstieg oder zur Aktivierung einer Proteinkinase C, mit weiteren Effekten in beiden Fällen. Second messenger sind intrazelluläre Botenmoleküle wie beispielsweise cAMP, cGMP, Ca²⁺ oder andere, welche über die Aktiverung oder Deaktivierung intrazellulärer Proteine Reaktionen in der Zelle auslösen.

Die heterotrimeren G-Proteine liegen an der Innenseite der Plasmamebran. Sie bestehen aus den drei Untereinheiten α, β und γ. Ein aktivierter Rezeptor nimmt Kontakt auf mit dem G-Protein-Heterotrimer, welches daraufhin in eine α-Untereinheit und den βγ-Komplex dissoziiert. Sowohl die aktivierte α-Untereinheit alsauch der βγ-Komplex können intrazelluläre Effektorproteine beeinflussen. Die G-Protein-α-Untereinheit Familie wird derzeit in vier verschiedene Klassen eingeteilt (Gαs-, Gαi-, Gαq- und Gα12-Klasse). GPCRs werden entsprechend des in die Signaltransduktion eingebundenen G-Proteins klassifiziert.
D.h., GPCRs der Gs-Klasse vermitteln über Aktivierung von Gαs die Stimulation der Adenylatcyclase und erhöhen die intrazelluläre cAMP-Konzentration,
GPCRs der Gi-Klasse vermitteln über Aktivierung von Gαi eine Hemmung der Adenylatzyklase und erniedrigen intrezelluläres cAMP GPCRs der Gq-Klasse vermitteln über Aktivierung von Gαq eine Stimulation verschiedener PLCβ-Isoformen und führen zu einer Hydrolyse von membranständigem Phosphatidyl-inositol-4.5-bisphosphat zu Diacylglycerol und Inositoltriphosphat (IP3). IP3 setzt aus intrazellulären Speichern Ca²⁺ frei.
Die meisten GPCRs können nur mit einer G-Protein-α-Untereinheit Familie Kontakt aufnehmen, d.h., sie besitzen Selektivität für einen bestimmten Signaltransduktionsweg. Für den Zweck ein Verfahren zu entwickeln, mit dessen Hilfe chemische Verbindungen identifiziert werden sollen, die GPCR abhängige Signaltransduktionswege modulieren, ist diese enge Spezifität sehr hinderlich. Darüberhinaus liefern nur solche Signaltransduktionswege ein geeignetes Signal , welches sich in einem Assaytyp mit hohem Probendurchsatz (= High Throughput Screening = HTS)) verwerten läßt, bei denen z.B. die G-Proteinaktivierung zum Anstieg des intrazellulären Ca²⁺-Spiegels führt.

Solche G-Proteine mit geänderter Rezeptorspezifität und unterschiedlicher Anbindung an einen Signaltransduktionsweg können durch Aneinanderfügen von Bestandteilen aus verschiedenen G-Proteinen zu Hybrid-G-Proteinen mittels der Methoden der Molekularbiologie und Biochemie konstruiert werden.
Hybrid-G-Proteine sind Fusionskonstrukte, die innerhalb eines Proteins Sequenzen verschiedener Gα-Untereinheiten vereinigen. So kann z.B. durch die Fusion der Rezeptorerkennungsregion von Gαi mit der Effektor-Aktivierungsregion von Gαq ein Gαq/i-Hybrid hergestellt werden, das Signale von Gi-gekoppelten Rezeptoren empfängt, aber den Gαq-PLCβ-Signaltransduktionsweg anschaltet. Ein derartiges Hybrid, bei welchem die C-terminalen 5 Aminosäuren von Gαq durch die entsprechende Gαi-Sequenz ersetzt worden ist (Gαqi5) wurde von Conklin et al., Nature 363, 274 - 276 (1993) erstmalig beschrieben.
Diese "Umkopplung" von Rezeptoren hat den Vorteil, daß der Assayendpunkt (Anstieg der intrazellulären Ca²⁺-Konzentration im Vergleich zur Hemmung der Adenylatcyclase) einfacher durch meßtechnische Verfahren zugänglich ist und im High-Throughput-Screening verwendet werden kann.
Nachteil dieser Gαq/Gαi Fusionskonstrukte ist jedoch, daß sie einige GPCRs wie z.B. den SSTR1-Rezeptor qi5 nicht aktivieren können (Conklin et al., Mol. Pharmacol. 50, 885 - 890 (1996)).
Ebenso wurden Fusionskonstrukte zwischen Gαq und Gαs beschrieben. Auch diese besitzen den Nachteil, daß sie nicht alle Gs-gekoppelten Rezeptoren wie beispielsweise den β2 adrenergen Rezeptor oder den Dopamin D1 Rezeptor an den PLCβ-Signaltransduktionsweg anbinden können.
Neben den C-terminalen Modifikationen zur Änderung der Anbindung von Rezeptoren an bestimmte Signaltransduktionswege wurde eine N-terminale Modifikation von Gαq beschrieben, die zur Rezeptorpromiscuität führt. Unter Rezeptorpromiskuität soll dabei die Fähigkeit eines G-Proteins verstanden werden, Signale von unterschiedlichen Rezeptoren aufnehmen und weiterleiten zu können. Es handelt sich um ein Gαq-Protein, bei welchem die hochkonservierte, N-terminale; 6aa umfassende Region deletiert wurde (Kostenis et al., J. Biol. Chem. 272, 19107 - 19110 (1997)). Diese Deletion erlaubt dem resultierenden Gq (auch -6q genannt), nicht nur Signale von Gqsondern auch von Gs- oder Gi/o-gekoppelten Rezeptoren zu empfangen und an PLCβ weiterzuleiten.
Diese Gα-Untereinheit erfaßt nun auch Rezeptoren wie den SSTR1-Somatostatin Rezeptor, den Dopamin D1 und den adrenergen β2-Rezeptor. Allerdings kann der Rezeptor edg5 auch durch dieses G-Protein nicht erfaßt werden. Darüberhinaus ist die Signalintensität dieses G-Proteins so schwach, daß es in der Praxis nicht verwendet werden kann (Kostenis et al., J. Biol. Chem. 272, 19107 - 19110 (1997)).
Weiterhin bekannt ist mit Gα16 eine Gα-Untereinheit, die GPCRs aus verschiedenen funktionellen Klassen an den PLCβ-Ca²⁺-Signaltransduktionsweg anbindet. Es handelt sich praktisch um ein von Natur aus unselektives G-Protein. Aber auch diese Untereinheit ist nicht universell einsetzbar, denn Rezeptoren wie der edg5-Rezeptor oder der SSTR1-Somatostatin Rezeptor koppeln nicht oder nur schwach an Gα16. Aus diesem Grunde wäre es von großem Nutzen, wenn ein G-Protein zur Verfügung stehen würde, welches durch weitere funktionellen Klassen der GPCRs aktiviert werden könnte und darüberhinaus in der Zelle ein ausreichend starkes Signal ergeben würde, welches in einem Assay insbesondere einem High Throuhput-Assay zur Identifizierung von Verbindungen, die GPCRs und/oder die jeweils abhängigen Signaltransduktionswege modulieren, verwertet werden könnte, wie z.B. die Erhöhung oder Erniedrigung der intrazellulären Ca²⁺-Konzentration.

WO 97/48820 und WO 99/05177 beschreiben nun Verfahren zur Identifizierung eines Modulators eines GPCRs, bei denen vermischte α-Proteine eingesetzt werden, z. B. gemäß WO 99/05177 ein mutiertes α-Protein der Gq-Klasse.

Die Aufgabe der vorliegenden Erfindung besteht demnach darin, weitere Hybrid-G-Proteine für Screeningverfahren zur Identifizierung von chemischen Verbindungen zur Verfügung zu stellen, die sich darin auszeichnen, daß sie eine sehr breite Spezifität hinsichtlich der erkennbaren GPCRs aufweisen und diese G-Proteine an einen Signalweg koppeln, der zu Erhöhung der intrazellulären Ca²⁺-Konzentration führt. Zusätzlich werden sie so hoch exprimiert, daß die Signalintensität verbessert wird.

Die Erfindung betrifft ein Verfahren zur Identifizierung einer chemischen Verbindung, welcher die Wirkung wenigstens eines von einem G-Protein gekoppelten Rezeptor (GPCR) abhängigen Signaltransduktionswegs eines biologischen Organismus modifizert,
wobei besagtes Verfahren folgende Verfahrensschritte enthält:
a) Bereitstellung mindestens einer Zelle enthaltend mindestens einen GPCR abhängigen Signaltransduktionsweg, in welcher ein oder mehr als ein G-Protein hergestellt wird;
b) Bereitstellung mindestens einer zu untersuchenden chemischen Verbindung;
c) In-Kontakt-Bringen einer Zelle gemäß a) mit einer zu untersuchenden chemischen Verbindung gemäß b);
d) Bestimmung des quantitativen oder qualitativen Effekts einer zu untersuchenden chemischen Verbindung aus b) auf den Signaltransduktionsweg einer Zelle aus a) mittels eines vom Signaltransduktionsweg abhängigen meßbaren Signals,
**dadurch gekennzeichnet, dass** mindestens ein G-Protein ausgewählt ist aus -6qi4myr oder -6qs5myr.

Die Modifizierung der Wirkung wenigstens eines von einem G-Protein gekoppelten Rezeptor (GPCR) abhängigen Signaltransduktionswegs eines biologischen Organismus kann inhibierend oder stimulierend erfolgen. Eine inhibierende Wirkung einer chemischen Verbindung liegt vor, wenn das vom Signaltransduktionsweg abhängige meßbare Signal in Anwesenheit der chemischen Verbindung schwächer ausfällt als wenn diese weggelassen wird. Verbindungen, die eine solche Wirkung hervorrufen, werden auch Antagonisten genannt. Andererseits liegt eine stimulierende Wirkung einer chemischen Verbindung vor, wenn das vom Signaltransduktionsweg abhängige meßbare Signal stärker ausfällt als wenn die chemische Verbindung weggelassen wird. Solche verbindungen werden auch Agonisten genannt.
Das Verfahren bedient sich in einer bevorzugten Ausführungsweise einer Zelle, in welcher wenigstens zwei G-Proteine hergestellt werden. Diese G-Proteine können von einem oder unterschiedlichen GPCRs abhängen. Grundsätzlich kommen zur Durchführung des Verfahrens alle geeigneten G-Proteine ungeachtet ihrer Rezeptorspezifität, ihrer Sequenz, ihres Aufbaus, der Herkunft bezüglich Zelle, Gewebe oder Organ oder bezüglich der Spezies, für die sie spezifisch sind, in Frage. Bevorzugt wird in der Zelle wenigstens ein G-Protein aus -6qi4myr, -6qs5myr, -6qi4,- - 6qs5 hergestellt. Bei den G-Proteinen -6qi4myr, -6qs5myr, -6qi4, -6qs5 handelt es sich um Hybrid-G-Proteine, die aus den Anteilen unterschiedlicher G-Proteine der Maus zusammengesetzt wurden und in einigen Fällen zusätzliche Modifikationen enthalten. Die G-Proteine können von der Zelle einzeln oder in Kombination hergestellt werden. Insbesondere kann von einer Zelle abgesehen von den bereits erwähnten Hybrid-G-Proteinen auch Galpha16 hergestellt werden. Jedes der G-Proteine kann einzeln oder in Kombination mit einem oder mehreren anderen G-Proteinen in einer Zelle vorliegen. Die Herstellung von Galpha16 in einer Zelle soll dabei aber immer so erfolgen, daß es nie alleine sondern in Kombination mit einem anderen der vorstehend erwähnten G-Proteine hergestellt wird.
Die Aminosäuresequenzen der G-Proteine sind offenbart für Gαq in Seq ID Nr. 2, für -6qi4myr in Seq ID Nr. 4, für-6qi4 in Seq ID Nr. 6 und Seq ID Nr. 8 und für Galpha16 in Seq ID Nr. 10.
Die Bereitstellung einer chemischen Verbindung erfolgt üblicherweise in gelöster Form. Bevorzugt wird zur Lösung der chemischen Verbindung Wasser verwendet. Die Lösung kann neben dem Lösungsmittel Puffersubstanzen, Salze oder Hilfsstoffe wie Lösungsvermittler, Detergentien, Konservierungsstoffe oder anderes enthalten

Die Bereitstellung einer Zelle umfaßt deren Herstellung, Anzucht und Weiterverarbeitung. Die Bereitstellung erfolgt beispielsweise durch Präparation geeigneten Zellmaterials aus Organen oder Geweben oder durch die Vermehrung von geeigneten Zelllinien oder Mikroorganismen. Für die Anzucht können verschiedene geeignete Nährmedien verwendet werden. Die Zellen werden bei der für den Organismus optimalen Temperaturgehalten. Gegebenenfalls werden dem jeweils verwendeten Wachstumsmedium Konservierunsmittel, Antibiotika, pH-Indikatoren, Blutserumbestandteile, Blutserum, Hilfsstoffe oder anderes zugegeben. Verfahren zur Herstellung, Anzucht und Weiterverarbeitung sind in Standardwerken beschrieben. (Beispiel: Basic Cell Culture; Ed. J.M. Davis; IRL Press; 1994).
Im vorstehend beschriebenen Verfahren wird in bevorzugten Ausführungsformen die Zelle einer Wirbeltierspezies, einer Insektenspezies, eines C. elegans oder einer Hefe bereitgestellt. In besonders bevozugten Ausführungsformen wird die Zelle einer HeLa-, 293-, COS-, CHO-Zelle oder Saccharomyces cerevisiae bereitgestellt.

Zur Bestimmung des quantitativen oder qualitativen Effekts einer zu untersuchenden chemischen Verbindung auf den Signaltransduktionsweg einer Zelle eines vom Signaltransduktionsweg abhängigen meßbaren Signals wird in einer bevorzugten Ausführungsform die intrazelluläre Ca²⁺-Konzentration verwendet werden. Die Änderung der intrazellulären Ca²⁺-Konzentration läßt sich beispielsweise durch Anwendung von Aequorin, einem Farbstoff, oder der FLIPR^{™}-Technologie der Firma "Molecular Devices" nachweisen.
Die wie vorstehen beschriebenen Verfahren können in einer bevorzugten Ausführungsform zur Identifizierung eines Arzneimittels verwendet werden.

Chemische Verbindungen, welche die Wirkung wenigstens eines von einem G-Protein gekoppelten Rezeptor (GPCR) abhängigen Signaltransduktionswegs eines biologischen Organismus modifizieren, können durch mindestens ein Verfahren dieser Erfindung identifiziert werden. Solche chemischen Verbindungen könnten beispielsweise Abwandlungen bezüglich der chemischer Struktur von hydrophilen Signalstoffen, die GPCRs induzieren, wie bestimmte Hormone, Geruchsstoffe , bestimmte Arzneimittel, umfassen.

Die vorliegende Offenbarung betrifft ferner eine Polynukleotidsequenz codierend für ein Polypeptid mit der Eigenschaft eines G-Proteins, wobei das Polypeptid aus einer der folgenden Sequenzen ausgewählt wird:
a) ein Polypeptid mit einer Aminosäuresequenz gemäß Seq ID Nr. 2 , Seq ID Nr. 4, Seq ID Nr. 6 oder Seq ID Nr. 8;
b) ein Polypeptid gemäß a), dem eine oder mehrere Aminosäuren fehlen;
c) ein Polypeptid gemäß a), bei dem eine oder mehrere Aminosäuren hinzugefügt sind;
d) eine allele Variante des Polypeptids gemäß a).

Die allelen Varianten umfassen alle Polypeptide, die sich aus der Basenzusammensetzung der charakteristischen Form des Gens am definierten Genlocus für die jeweiligen Anteilspartner der Hybridproteine ergeben.
Die Offenbarung betrifft außerdem ein Polynukleotid enthaltend eine Polynukleotidsequenz, wobei die Polynukleotidsequenz aus einer der folgenden Sequenzen ausgewählt wird:
a) eine Polynucleotidsequenz gemäß Seq ID Nr. 1, Seq ID Nr. 3, Seq ID Nr. 5 oder Seq ID Nr. 7 oder deren entsprechende komplementäre Sequenz;
b) eine Polynukleotidsequenz, die unter stringenten Bedingungen mit einer Polynukleotidsequenz gemäß a) hybridisiert.

Die Stringenz einer Lösung wird bestimmt durch deren Temperatur und Salzgehalt. Mit der Stringenz kann das Ausmaß der Basenpaarung zweier homologer Nukleotidsequenzen eingestellt werden. Die Stringenz ist abhängig von der Länge und der Basenzusammensetzung eines Polynukleotids. Stringente Bedingungen im Sinne dieser Erfinduing liegen vor, wenn die Polynukleotidsequenz und die hybridisierende Sequenz eine Übereinstimmung von 95% oder mehr aufweise

Eine bevorzugte Ausführungsform einer Polynukleotidsequenz bzw. eines Polynucleotids wie vorstehend beschrieben ist ein Polynukleotid, welches Bestandteil einer rekombinanten Vektorkonstruktion ist. Rekombinante Vectorkonstruktionen können unter Zuhilfeneahme des einschlägigen Fachwissens, wie dargestellt beispielsweise in "F.M. Ausubel et al., Current Protocols in Molecular Biology, Wiley &Sons, New York", hergestellt werden. Dabei wird ein Polynukleotid codierend für eine Aminosäuresequenz gemäß einer der im vorhergehenden beschriebenen Sequenzinformationen (Seq ID Nr. 2, 4, 6, 8) oder eine Polynukleotidsequenz gemäß einer der im vorhergehenden beschriebenen Sequenzinformationen (Seq ID No. 1, 3, 5, 7) in einen Basisvektor eingebaut. Als Basisvektor soll ein Vektor verstanden werden, in welchen eine Polynucleotidsequenz eines Polynucleotids mittels der Methoden der Molekularbiologie eingebaut und in einem Mikroorganismus beispielsweise einem Bakterium, Pilz oder der Zelle einer Zellkultur kloniert werden kann. Der Basisvektor kann beispielsweise aus einem Plasmid mit einem Antibiotikumresistenzmarker, einem "origin of replication" geeignet zur Vermehrung des Plasmids in Bakterien oder Zellkulturen, sowie einem Promotor geeignet zur Expression eines Proteins bestehen. Der Basisvektor kann beispielsweise auch aus einem Phagenvektor, einem Phagemidvektor, einem Phasmidvektor, einem Cosmidvektor, einem Virusvektor, einem YAC-Vektor oder anderen Vektortyp bestehen. Beispiele für Basisvektoren sind pUC 18, pUC19, pBluescript, pKS, pSK und andere. Der Einbau des einzubauenden Polynukleotids erfolgt über geeignete Restriktionsschnittstellen mittels der entsprechenden Restriktionsenzyme, welche kommerziell von Firmen wie BioLabs, Roche Diagnostics, Stratagene und anderen angeboten werden. Solche Restriktionsschnittstellen können beispielsweise die Erkennungsstellen der Restriktionsenzyme BamHl, EcoRl, Sall, EcoRV und andere sein.

Die rekombinante Vektorkonstruktion besteht in einer bevorzugten Ausführungsform aus einem in Eukaryonten und/oder Prokaryonten verwendbaren Expressionsvektor. Ein Expressionsvektor enthält einen Promotor, der funktionell mit einer Polynukleotidsequenz verbunden werden kann, so daß ein von dieser Polynukleotidsequenz codiertes Protein in einem biologischen Organismus beispielsweise einem Bakterium, Pilz oder der Zelle einer eukaryotischen Zellliniesynthetisiert wird.. Der Promotor kann induzierbar sein beispielsweise mittels Tryptophan oder er kann konstitutiv aktiv sein.. Beispiele für Expressionsvektoren sind pUC18, pUC19, pBluescript, pcDNA3.1 oder andere.

Die vorliegende Offenbarung bezieht sich weiterhin auf eine Wirtszelle, welche ein Polynucleotid oder eine wie vorstehend beschriebene rekombinante Vektorkonstruktion enthalten kann. Die Wirtszelle besteht in einer bevorzugten Ausführungsform aus einer humanen Zelle. In weiteren bevorzugten Ausführungsformen besteht die Wirtszelle aus der Zelle einer Wirbeltierspezies, einer Insektenspezies, eines C. elegans, eines Bakterium oder einer Hefe. In besonders bevorzugten Ausführungsformen besteht die Zelle aus einer HeLa-, 293-, COS-, CHO-Zelle, der Zelle eines Escherichia coli oder der Zelle eines Saccharomyces cerevisiae. Darüberhinaus können andere eukaryotische Zellen insbesondere Zelllinien, Bakterien insbesondere Bacillus spec., Streptomyces spec. oder Pilze insbesondere Penicillium spec, Aspergillus spec. Verwendet werden.

Die Offenbarung betrifft weiterhin die Herstellung einer wie vorstehend beschriebenen Wirtszelle durch Einführung eines Polynuleotids gemäß einem oder mehreren der Polynukleotidsequenzen wie offenbart in Seq ID Nr. (1 - 8) oder einer wie zuvor charakterisierten rekombinanten Vektorkonstruktion in eine eukaryotische oder prokaryotische Zelle. Die Einführung der Polynukleotidsequenzen kann beispielsweise durch Elektroporation oder Transformation der eukaryotischen oder prokaryotischen Zellen mit der Polynukleotidsequenz, weiterhin durch Ca²⁺-Phosphat-Präzipitation der eukaryotischen oder prokaryotischen Zellen zusammen mit der Polynukleotidsequenz oder anderen Methoden erfolgen.

Eine solche Wirtszelle kann zur Durchführung eines vorstehend beschriebenen Verfahrens dieser Erfindung verwendet werden.

Darüberhinaus bezieht sich die vorliegende Offenbarung Darüberhinaus bezieht sich die auf ein Verfahren zur Herstellung eines Proteins bestehend aus einer Aminosäuresequenz ausgewählt aus einer der Sequenzen Seq ID Nr. 2, Seq ID Nr. 4, Seq ID Nr. 6, Seq ID Nr. 8 enthaltend folgende Verfahrensschritte:
a) Herstellung einer Wirtszelle enthaltend eine entsprechende Polynukleotidsequenz und hergestellt wie im vorhergehenden beschrieben;
b) Anzucht dieser Wirtszelle in einem für die Wirtszelle geeigneten Wachstumsmedium sowie eventuell die Induktion der Expression des Proteins;
c) Gewinnung des Zellmaterials und Aufschluß der Zellen;
d) Abtrennung eines Proteins mittels biochemischer Methoden zur Proteinreinigung.

Für die Herstellung und Reinigung der bezeichneten Proteine können bekannte Methoden wie beschrieben in "F.M. Ausubel et al., Current Protocols in Molecular Biology, Wiley &Sons, New York" entsprechend verwendet werden.

### Beispiele

### Beispiel 1:

### Aktivierung eines Signaltransduktionswegs durch die Gα-Protein-Mutante -6q durch verschiedene Rezeptoren

COS7 Zellen wurden in DMEM (Dulbeccos's modified Eagle's Medium) unter Zusatz von 10% FCS (Fötales Kälberserum) bei 37°C (5%CO₂) kultiviert. Für Transfektionen wurden 1 x 10⁶ Zellen in 100-mm Platten ausgesät. Etwa 24 h später wurden die Zellen mit den Expressionsplasmiden αq oder -6q (1 µg DNA/100 mm Platte) und jeweils eines der folgenden folgenden Rezeptorkonstrukte (4 µg DNA/100 mm Platte) kotransformiert: M2 (Muskarinrezeptor in pCD), D2 (Dopamin Rezeptor in pCDNAI), kappa (Opioid Rezeptor in pCDNA3), SSTR1 (Somatostatin-Rezeptor in pCMV), A1 (Adenosin Rezeptor in CDM7), D1 (Dopamin Rezeptor in pCDNAI), V2 (Vasopressin Rezeptor in pCD-ps), β2 (adrenerger Rezeptor in pSVL).

Etwa 24 h nach der Transfektion wurden die Zellen in 6well Platten in gleichen Portionen aufgeteilt und in DMEM 3 µCi/ml [³H]myo-inositol (20Ci/mmol) zugegeben. Nach 24 stündiger Inkubation wurden die Zellen bei Raumtemperatur mit HBSS (+ 10 mM LiCI) für 20 min inkubiert. Danach wurden die Zellen für eine Stunde mit den entsprechenden Agonisten stimuliert und der Anstieg der intrazellulären Inositolmonophosphate durch Anionen Austausch Chromatographie bestimmt.
Der Gα-Protein-Mutanten -6q fehlen verglichen mit der Wildtypsequenz die sechs hochkonservierten Aminosäurereste des aminoterminalen Endes. Die Ergebnisse im folgenden wurden mit der Gα-Protein Konstruktion -6q erhalten. Die Herstellung solcher Mutanten oder der verwendeten Rezeptorkonstrukte erfolgte mit Hilfe von Standardmethoden der Molekularbiologie wie ausführlich beschrieben beispielsweise in "F.M. Ausubel et al., Current Protocols in Molecular Biology, Wiley & Sons, New York".

COS7 Zellen, die WTq oder -6q und verschiedene Gi/o- (A) oder Gs-gekoppelte Rezeptoren (B) exprimieren wurden für 1 h (37°C) in An- und Abwesenheit der entsprechenden Agonisten (s.u.) inkubiert. Der Anstieg der intrazellulären IP1 Konzentration wurde wie im beigefügten Protokoll 1 gemessen. Die Daten stellen Mittelwerte + S.E. von 3-7 unabhängigen Experimenten dar, jeweils als Dreifachbestimmungen ausgeführt. Die folgenden Liganden wurden benutzt: A m2 (Muskarin Rezeptor): Carbachol (100µM); D2 (Dopamin Rezeptor): (-)-Quinpirole (10µM); kappa (Opioid Rezeptor): (-)-U50488 (10µM); SSTR1 (Somatostatin Rezeptor): Somatostatin14 (1µM); B, A1 (Adenosin Rezeptor): R(-)-PIA (10mM);
B D1 (Dopamin Rezeptor): Dopamin (1 mM); V2 (Vasopressin Rezeptor): AVP (1 nM); β2 (adrenerger Rezeptor): (-)-Isoproterenol (200µM).
Es wurde ersichtlich, daß die Gα-Protein-Mutante -6q eine IP1-Bildung in Abhängigkeit von unterschiedlichen Rezeptorklassen stimuliert. 1P1 ist ein Signalmolekül, welches im PLC-β-Signaltransduktionsweg entsteht und im weiteren Verlauf der Signalweitergabe zur Erhöhung der intrazellulären Ca²⁺-Konzentration führt. Die IP1- Freisetzung mittels des -6q-Konstrukts gelingt sowohl mit Gi/o gekoppelten (A: m2, D2, k-OR, SSTR1, A1) als auch mit Gs gekoppelten (B: D1, V2, β2) Rezeptoren.

### Beispiel 2:

### Herstellung hochexprimierender Mutanten von Gα-Proteinen mit breiter Rezeptorspezifität

Zuerst wurden hybride G-Protein α-Untereinheiten konstruiert, denen die sechs hochkonservierten Aminosäuren des Aminoterminus fehlen und die gleichzeitig am C-terminus entweder eine αi oder αs-Sequenz aufweisen. Entsprechend der enthaltenen αi-Sequenz bzw. αs-Sequenz erfolgt die Bezeichnung als -6qi4 oder - 6qs5. Das Konstrukt -6qi4 verbindet die Gs- sowie einige Gi/o-gekoppelte Rezeptoren mit dem PLCβ-Signaltransduktionsweg. Diese Rezeptoren erfaßen auch den SSTR1- oder den edg5-Rezeptor. Der edg5-Rezeptor läßt sich mit Gα16 nicht an den PLCβ-Signaltransduktionsweg anbinden. Gα16 ist ein G-Protein mit breiter Rezeptorspezifität, weicher in WO 97/48820 (Titel: Promiscuous G-protein compositions and their use) offenbart ist.
Das Konstrukt -6qs5 bindet die Gi/o- sowie noch zusätzliche Gs-gekoppelte Rezeptoren an den PLCβ-Signaltransduktionsweg und erfaßt nun auch Rezeptoren wie den Dopamin D1 oder den adrenergen β2-Rezeptor.
Eine Kombination dieser beiden G-protein α-Untereinheiten in einer Zelllinie erfaßt damit ein weiteres Spektrum an GPCRs als jede Untereinheit für sich oder Gα16.

Die Anwendbarkeit in technischen Screeningverfahren ließe sich weiter verbessern, wenn die Expression dieser Gα-Untereinheiten (-6qi4; -6qs5) erhöht würde, weil damit auch ein stärkeres Signal erzielt werden könnte.
Aus diesem Grunde wurden zusätzliche Myristoylierungs/Palmitoylierungs-Erkennungssequenzen in den aminoterminalen Bereich der Gα-Untereinheiten eingebaut. Dies führte zur Konstruktion der G-Proteine -6qi4myr und -6qs5myr. Die Proteinsequenz von -6qi4myr und -6qs5myr bezüglich des Aminoterminus ist MGCC im Unterschied zu MACC der Ausgangssequenz der-6q-Varianten. Die neuen Konstrukte -6qi4myr und -6qs5myr enthalten nun eine Konsensus-Sequenz für die Myristoylierung/Palmitoylierung. -6qi4myr wurde aus -6qi4 und -6qs5myr wurde aus -6qs5 hergestellt. Es ist bekannt, daß die Enfernung von Myristyl- oder Palmitylresten in G-Proteinen zu einer Umverteilung in der Zelle führen: Verlust von Palmitat- oder Myristatresten beeinflussen das Expressionsmuster der G-Proteine in der Weise, daß Wegnahme der Fettsäurereste eine hauptsächlich zytosolische Lokalisation zur Folge hat. G-Protein α-Untereinheiten findet man sowohl in der Zellmembran als auch im Cytosol. Jedoch können nur die membranständigen G-Proteine die Signale der GPCRs an die intrazellulären Effektoren weiterleiten. Bekannt waren nur die Konsequenzen der Entfernung einer Konsensussequenz für Palmitoylierung/Myristoylierun durch Mutation. Nicht bekannt hingegen war, daß die Einführung der zusätzlichen Konsensusstelle für die Myristoylierung/Palmitoylierung in den Gα-Deletionsmutanten zur Erhöhung der Expression führt. Es konnte gezeigt werden, daß die Einführung zusätzlicher Palmitoylierungs/Myristoylierungsstellen die in der Zellmembran exprimierte Menge an Gα-Untereinheit steigert. Im SDS-PAGE-Westernblot (Natrium-Dodecylsulfat Polyacrylamidgelelektrophorese-Westernblot) ist zu erkennen, daß die Expression von -6qi4myr im Vergleich zu -6qi4 deutlich erhöht ist. Ein SDS-PAGE-Westernblot einer Fraktionierung in partikuläre Fraktion (p; Membranen enthaltend) und lösliche Fraktion (s; sc) von qwt und -6qi4myr zeigt, daß die höher myristoylierte/palmitoylierte Variante -6qi4myr nur in der partikulären Fraktion enthalten ist.
Jeweils 20 µg Membranproteine wurden dazu aus transfizierten COS7 Zellen prepariert, durch SDS-PAGE Gelelektrophorese (10%) aufgetrennt und mittels Westernblot Analyse unter Verwendung des 12CA5 monoklonalen Antikörpers (Roche Biosciences) analysiert.
Alle G-Protein α Untereinheiten wurden mit dem 12CA5 monoklonalen Antikörper (gekoppelt an horseradish-peroxidase), der gegen den HA-epitop tag gerichtet ist, detektiert. Der HA-tag ist in allen G-Protein Konstrukten enthalten. In qwt und qi5 ersetzt er die Aminosäuren 125-130, in den N-terminal deletierten G-Proteinen (-6q, - 6qi4, -6qi4myr) die Aminosäuren 119-124. Jeweils 20 µg Membranprotein, prepariert aus transfizierten COS7 Zellen wurden mittels SDS PAGE Geietektrophorcse aufgetrennt, auf Nitrocellulose geblottet und die G-Protein α Untereinheiten mit dem 12CA5 Antikörper detektiert. Immunoreaktive G-Proteine wurden mit einem Chemilumineszenzsystem (Amersham) sichtbar gemacht.

### Beispiel 3 :

### Stimulierung verschiedener hoch exprimierter Gα-Proteine mit breiter Rezeptorspezifität durch unterschiedliche Rezeptoren

Die Stimulierung der hoch exprimierten Gα-Varianten -6qs5myr und -6qi4myr durch unterschiedliche Rezeptoren wurde untersucht. Es war zu erkennen, daß -6qi4myr (=Δ6qi4myr) durch Gi/o-gekoppelter Rezeptoren (beispielsweise Dopamin D2, edg5, CCR5, SSTR1, KOR) an den PLCβ-Signaltransduktionsweg angeschlossen wird und zu einem hohen Signal führt, welches proportional der Ca²⁺-Freisetzung verläuft. Als Kontrollen wurden ein Vektorkonstrukt sowie das Gα16-Protein (+16) verwendet. Es wurde gezeigt, daß Gs-gekoppelte Rezeptoren durch -6qs5myr (=Δ6qs5myr) an den PLCβ-Signaltransduktionswegangebunden werden. Als Referenz dient auch hier das G-Protein Gα16.
Zur experimentellen Bestimmung des freigesetzten Ca²⁺ mittels des Aequorinsystems wurden CHO Zellen kotransfiziert mit dem Apoaequorin Expressionsplasmid cytAEQ/pCDNAI, der angegebenen Rezeptor DNA (SSTR1, KOR, D2, D1, beta2) sowie den G-Protein α Untereinheiten G16 und -6qi4myr unter Verwendung von Lipofectamin. Nach 10stündiger Inkubation in OPTIMEM Medium wurden die Zellen einmal mit RPMI 1640 Medium gewaschen und mit 5 µM Coelenterazine f in RPMI 1640 für 2 h bei 37°C inkubiert. Danach wurden die Zellen zweimal mit PBS gewaschen und mit den entsprechenden Rezeptoragonisten stimuliert: Somatostatin 14 für den SSTR1 Rezeptor, U50488 für den kappa Opioid Rezeptor, (-)-Quinpirol für den Dopamin D2 Rezeptor, Dopamin für den Dopamin D1 Rezeptor und Isoproterenol für den beta2-Rezeptor. Agoniststimulation der Gi/o-gekoppelten Rezeptoren (SSTR1, KOR, D2) und der Gs-gekoppelten Rezeptoren (D1, beta2) führt zu Aktivierung der Gproteine G16 sowie -6qi4myr mit nachfolgender Stimulation der PLCβ und intrazellulärer Ca-Freisetzung. Ca-Bindung an den Apoaequorin-Coelenterazine Komplex führt zu Lichtemission, welche mit einem Luminometer (TOPCount, Hewlett Packard) gemessen wurde.

### Zusammenfassung der Ergebnisse:

1. Alignement der aminoterminalen Bereiche verschiedener Gα-Proteine.
2. Stimulation des PLCβ-Signaltransduktionswegs mittels der-6q-Gα-Proteinvariation durch Gi/o gekoppelte (A) und Gs gekoppelte (B) Rezeptoren unter Verwendung der höchstmöglichen Konzentration des jeweiligen Agonisten.
3. Erhöhung der Expression von -6qi4myr im Vergleich zu -6qi4 gemäß SDS-PAGE-Westernblot. Daneben ist die Expression weiterer Gα-Proteine gezeigt.
4. Fraktionierung in partikuläre Fraktion (p; Membranen enthaltend) und lösliche Fraktion (s; sc) von qwt und -6qi4myr gemäß SDS-PAGE-Westernblot. Die G-Protein Untereinheiten wurden mit dem 12CA5 monoklonalen Antikörper detektiert und ergeben Proteinbanden in einer Größe von - 42KD.
5. Anbindung verschiedener Gi/o-gekoppelter Rezeptoren durch - 6qi4myr (=Δ6qi4myr) an den PLCβ-Signaltransduktionsweg. D2, Kappa und SSTR1 sind Gi/o-gekoppelte Rezeptoren. Als Kontrollen wurden ein Vektorkonstrukt sowie das Gα16-Protein (+16) verwendet.
6. Anbindung Gs-gekoppette Rezeptoren durch -6qs5myr (=Δ6qs5myr) an den PLCβ-Signaltransduktionsweg. β1, β2 und D1 sind Gs-gekoppelte Rezeptoren. Als Referenz dient ein Vektorkonstrukt und das G-Protein Gα16 (+16).
7. Anbindung des Gi/o-gekoppelten Dopamin D2 Rezeptors an den PLCβ-Ca-Signaltransduktionsweg in Gegenwart der wenig empfindlichen α-Untereinheit G16, in Gegenwart der sehr sensitiven G α-Untereinheit -6qi4myr sowie in Kombination von G16 und -6qi4myr. Es ist offensichtlich, daß die potente Aktivierung der Calcium Freisetzung durch -6qi4myr durch die Anwesenheit von G16 nicht beeinträchtigt wird.

## Patentansprüche

1. Verfahren zur Identifizierung einer chemischen Verbindung, welcher die Wirkung wenigstens eines von einem G-Protein gekoppelten Rezeptor (GPCR) abhängigen Signaltransduktionswegs eines biologischen Organismus modifiziert, wobei besagtes Verfahren folgende Verfahrensschritte enthält:
a) Bereitstellung mindestens einer Zelle, enthaltend mindestens einen GPCR abhängigen Signaltransduktionsweg, in welcher ein oder mehr als ein G-Protein hergestellt wird;
b) Bereitstellung mindestens einer zu untersuchenden chemischen Verbindung;
c) In-Kontakt-Bringen einer Zelle gemäß a) mit einer zu untersuchenden chemischen Verbindung gemäß b);
d) Bestimmung des quantitativen oder qualitativen Effekts einer zu untersuchenden chemischen Verbindung aus b) auf den Sighaitransduktionsweg einer Zelle aus a) mittels eines vom Signaltransduktionsweg abhängigen messbaren Signals,
**dadurch gekennzeichnet, dass** mindestens ein G-Protein ausgewählt ist aus -6qi4myr oder -6qs5myr, wobei "-6qi4" bedeutet, dass die G-Protein-α-Untereinheit der Klasse Gαq eine N-terminale Deletion von 6 Aminosäuren trägt und am C-Terminus eine αi-Sequenz aufweist, und wobei "6qs5" bedeutet, dass die G-Protein-α-Untereinheit der Klasse Gαs eine N-terminale Deletion von 6 Aminosäuren trägt und am C-Terminus eine αs-Sequenz aufweist, und "myr" bedeutet, dass das jeweilige G-Protein am Aminoterminus eine MGCC Myristoylierungs/Palmitoylierungs-Erkennungssequenz anstelle des Gαq spezifischen Sequenz MACC aufweist.

2. Verfahren nach Anspruch 1, wobei in der Zelle, welche gemäß a) bereitgestellt wird, wenigstens zwei G-Proteine hergestellt werden.

3. Verfahren nach Anspruch 1 oder 2, wobei in der Zelle, welche gemäß a) bereitgestellt wird, wenigstens zwei G-Proteine ausgewählt aus -6qi4myr, 6qs5myr, -6qi4, -6qs5 oder Galpha16 hergestellt werden.

4. Verfahren nach einem oder mehreren der Ansprüche 1 bis 3, wobei die Zelle gemäß a) die Zelle einer Wirbeltierspezies, einer Insektenspezies, eines C. elegans oder einer Hefe ist.

5. Verfahren nach Anspruch 4, wobei die Zelle eine HeLa-, 293-, COS-, CHO-Zelle oder eine Zelle von Saccharomyces cerevisiae ist.

6. Verfahren nach einem oder mehreren der Ansprüche 1 bis 5, wobei zur Bestimmung des quantitativen oder qualitativen Effekts einer zu untersuchenden chemischen Verbindung mittels eines vom Signaltransduktionsweg abhängigen messbaren Signals gemäß Anspruch 1 d) die intrazelluläre Ca²⁺ -Konzentration verwendet wird.

7. Verfahren zur Identifizierung eines Arzneimittels gemäß einem oder mehreren der Ansprüche 1 bis 6.

## Claims

1. A process for identifying a chemical compound modifying the action of at least one G-protein-coupled receptor (GPCR)-dependent signal transduction pathway of an organism, wherein said process comprises the following process steps:
a) providing at least one cell which contains at least one GPCR-dependent signal transduction pathway and which produces one or more than one G-protein;
b) providing at least one chemical compound to be studied;
c) contacting a cell according to a) with a chemical compound to be studied according to b) ;
d) determining the quantitative or qualitative effect of a chemical compound to be studied from b) on the signal transduction pathway of a cell from a) by means of a signal transduction pathway-dependent measurable signal,
which process comprises at least one G-protein being selected from -6qi4myr or -6qs5myr, with "-6qi4" meaning that the G-protein α subunit of the Gαq class carries an N-terminal deletion of 6 amino acids and has an αi sequence at the C terminus, and with "-6qs5" meaning that the G-protein α subunit of the Gαs class carries an N-terminal deletion of 6 amino acids and has an αs sequence at the C terminus, and with "myr" meaning that the particular G-protein has an MGCC myristoylation/palmitoylation recognition sequence rather than the Gαq-specific MACC sequence at the amino terminus.

2. The process as claimed in claim 1, wherein the cell provided according to a) produces at least two G-proteins.

3. The process as claimed in claim 1 or 2, wherein the cell provided according to a) produces at least two G-proteins selected from -6qi4myr, -6qs5myr, -6qi4, -6qs5 or Galpha16.

4. The process as claimed in one or more of claims 1 to 3, wherein the cell according to a) is the cell of a vertebrate species, an insect species, a C. elegans or a yeast.

5. The process as claimed in claim 4, wherein the cell is a HeLa, 293, COS or CHO cell or a cell of Saccharomyces cerevisiae.

6. The process as claimed in one or more of claims 1 to 5, which comprises using the intracellular Ca²⁺ concentration for determining the quantitative or qualitative effect of a chemical compound to be studied by means of a signal transduction pathway-dependent measurable signal as claimed in claim 1 d).

7. A process for identifying a pharmaceutical as claimed in one or more of claims 1 to 6.

## Revendications

1. Procédé d'identification d'un composé chimique, qui modifie l'action d'au moins une voie de transduction de signaux dépendant d'un récepteur couplé à une protéine G (GPCR) d'un organisme biologique, ledit procédé contenant les étapes de procédé suivante
a) mise à disposition d'au moins une cellule contenant au moins une voie de transduction de signaux dépendant d'un GPCR, dans laquelle une ou plus d'une protéine G est produite ;
b) mise à disposition d'au moins un composé chimique à analyser ;
c) mise en contact d'une cellule selon a) avec un composé chimique à analyser selon b) ;
d) détermination de l'effet quantitatif ou qualitatif d'un composé chimique à analyser de b) sur la voie de transduction de signaux d'une cellule de a) au moyen d'un signal mesurable dépendant d'une voie de transduction de signaux,
**caractérisé en ce qu'**au moins une protéine G est choisie parmi -6qi4myr ou -6qs5myr, où "-6qi4" signifie que la sous-unité α de la protéine G de la classe Gαq porte une délétion N-terminale de 6 acides aminés et présente en l'extrémité C une séquence αi et où "-6qs5" signifie que la sous-unité α de la protéine G de la classe Gαs porte une délétion N-terminale de 6 acides aminés et présente en l'extrémité C une séquence αs, et "myr" signifie que la protéine G présente en l'extrémité amino une séquence de détection myristoylation/palmitoylation de MGCC au lieu d'une séquence MACC spécifique de Gαq.

2. Procédé selon la revendication 1, dans lequel au moins deux protéines G sont produits dans la cellule qui est mise à disposition selon a).

3. Procédé selon la revendication 1 ou 2, dans lequel au moins deux protéines G choisies parmi -6qi4myr, -6qs5myr, -6qi4, -6qs5 ou Galpha16 sont produites dans la cellule qui est mise à disposition selon a).

4. Procédé selon l'une ou plusieurs des revendications 1 à 3, dans lequel la cellule selon a) est la cellule d'une espèce de vertébré, d'insecte, d'un C. elegans ou d'une levure.

5. Procédé selon la revendication 4, dans lequel la cellule est une cellule HeLa, 293, COS, CHO ou une cellule de Saccharomyces cerevisiae.

6. Procédé selon l'une ou plusieurs des revendications 1 à 5, dans lequel on utilise la concentration intracellulaire en Ca²⁺ pour la détermination de l'effet quantitatif ou qualitatif d'un composé chimique à analyser au moyen d'un signal mesurable selon la revendication 1 d) dépendant d'une voie de transduction de signaux.

7. Procédé pour l'identification d'un médicament selon l'une ou plusieurs des revendications 1 à 6.
